# EUROPEAN PATENT SPECIFICATION

(11) **EP 0 948 934 B1**
(45) Date of publication and mention of the grant of the patent: **26.11.2003**
(21) Application number: 99250234.4
(22) Date of filing: 04.02.1994
(51) Int. Cl.: A61B 17/06

(54) **Surgical needle**
Chirurgische Nadel
Aiguille chirurgicale

(30) Priority: 13.02.1993 DE 4304739
(43) Date of publication of application: 13.10.1999
(62) Divisional of application: 94250021.6
(73) Proprietor: ETHICON INC., Somerville, New Jersey 08876-0151 (US)
(72) Inventor: Brunken, Dieter, 24641 Hüttblek (DE)
(74) Representative: UEXKÜLL & STOLBERG

(56) References cited:
- EP-A- 0 263 347
- EP-A- 0 296 776
- DE-C- 3 841 443
- DE-C- 4 208 242
- US-A- 3 840 015
- PATENT ABSTRACTS OF JAPAN vol. 13, no. 286 (C-613) [3634], 29 June 1989 (1989-06-29) & JP 01 083252 A (KAIHATSU), 29 March 1989 (1989-03-29)

## Description

The invention relates to a surgical needle with a suture material or thread secured to the end opposite the puncture tip.

Such surgical needles are generally known and in most cases consist of a corrosion-resistant metal, preferably of chrome-nickel steel. With such needles, whose whole length is bare or has not been surface-treated, precise establishment of the puncture point and estimation of the puncture depth is frequently not unproblematical during an operation. In addition, when pulling out the needle after the tissue has been pierced, determination of how much further the needle must still be pulled until its rear section with the thread attachment has also emerged from the tissue is in many cases possible only with difficulty.

Known from U.S. Patent No. 3 840 015 is a surgical needle whose tip is provided with a photoluminescent covering which, when irradiated by a suitable source, is excited to light up. Otherwise the needle is bare or untreated at the surface. The photoluminescent covering improves the visibility of the needle tip, which is a help in establishing the puncture point and greatly facilitates the use of the needle. A disadvantage is that an additional radiation source is necessary to cause the photoluminescent covering to light up. Moreover, such a covering is not unproblematical in terms of its toxicity.

The object of the invention is to provide a surgical needle which can be handled more easily and which makes more precise operation possible.

This object is achieved by a surgical needle with the features of claim 1. Advantageous versions result from the dependent claims.

With the inventive surgical needle, the surface of the puncture tip and of the zone of the needle adjoining same consists of bare or untreated metal, while the surface of the remaining section of the needle, which can account for up to ca. 50% of the needle length, is matt-finished or coloured, continuously or with small breaks, by chemical or electrolytic means or by a covering, up to the thread attachment. Because of the surface treatment of the rear section, this stands in clear contrast to the remainder of the suture during operation. This makes it easier for the surgeon to grasp the needle quickly and surely in the right place with a needle holder. The surgical needle according to the invention also serves as a marking carrier, and the surgeon can, by noting the length of the front zone of the rear section of the needle which, when the tissue is pierced, has already emerged on the other side of the tissue, tell quickly and surely how much further he still has to pull the needle until it has completely pierced the tissue.

The section of the needle subjected to a surface treatment need not be continuously coloured or matt-finished, but can for example also be matt-finished or coloured in annular or speckled form, the surface between the rings or speckles being bare or untreated. The intermediate spaces lying bare in the zone which is matt-finished or coloured with breaks are preferably not to be larger than 1 to 2 mm. This gap is enough to create a closed impression of the rear section of the needle. On the other hand, the bare areas can improve the visibility or conspicuousness of the corresponding needle zone.

The invention will be explained in more detail below with the help of embodiments. The drawings show:
- Figure 1: a magnified representation of a surgical needle according to the invention in which the rear section is matt-finished or coloured in speckled form,
- Figure 2: a magnified representation of another surface treatment pattern in which the surface is continuously matt-finished or coloured, and
- Figure 3: a magnified representation of still another surface treatment pattern in which the surface is matt-finished or coloured in annular form.

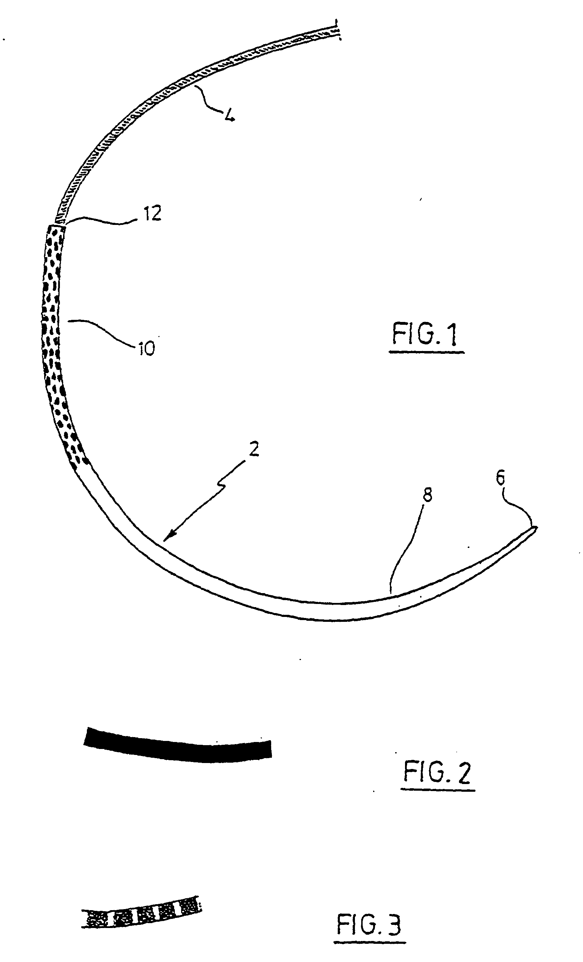

The needle 2 shown in Figure 1 is a normal semi-circular round-bodied needle; the needle can, however, have any desired form and be, for example, a blunt round-bodied needle, a cutting needle or a spatula needle. Located at one end of the needle 2 is the puncture tip 6, which can also be designed as a microtip, while a thread attachment 12, where a thread 4 made from surgical suture material is secured, is formed at the opposite end of the needle 2.

In the version of the needle 2 shown in Figure 1, the surface of the puncture tip 6 and of the zone 8 adjoining same consists of bare or untreated metal. The surface of the remaining or rear section 10 is matt-finished or coloured (by chemical or electrolytic means or by a covering) in the form of speckles. The gaps between the speckles are not to exceed 1 to 2 mm. The remaining section 10 of the needle 2 accounts for less than 50% of the needle length, preferably for less than 25%.

A suitable colouring or matt-finishing can be,achieved either by chemical means through pickling or etching, or electrolytically through appropriate anodic or cathodic treatment, where appropriate with polarity inversion or through alternating current. A special form of pickling is so-called dipping or matt-dipping. Stoving lacquers can also be used for colouring or matt-finishing.

The rear section of the needle can also be continuously matt-finished or coloured, i.e. having the "pattern" shown in Figure 2.

Figure 3 shows a version of the surface treatment pattern in which the surface is matt-finished or coloured in annular form, whereby the matt-finished or coloured rings, which can be of any width, are ca. 1 to 2 mm apart.

With all the needles shown, the colour in the matt-finished or coloured zones can agree with the colour of the thread 4, which offers the additional advantage that the coding colour of the thread is intuitively detectable when needles are in packs.

## Claims

1. Surgical needle with a suture material or thread (4) secured to the end opposite the puncture tip (6), **characterized in that** the surface of the puncture tip (6) and of the zone (8) of the needle (2) adjoining same, extending over more than ca. 50% of the length of the needle, consists of bare or untreated metal, while the surface of the remaining section (10) of the needle (2) is matt-finished or coloured, continuously or with small breaks, by chemical or electrolytic means or by a covering, up to the thread attachment (12).

2. Surgical needle according to claim 1, **characterized in that** the zone (10) of the needle (2) whose surface is matt-finished or coloured, continuously or with small breaks, by chemical or electrolytic means or by a covering, accounts for less than 25% of the needle length.

3. Surgical needle according to one of the previous claims, **characterized in that** the matt-finished or coloured zone of the needle is matt-finished or coloured in annular form or in speckled form (10), the surface between the rings or speckles being bare or untreated.

## Patentansprüche

1. Chirurgische Nadel mit einem an dem der Einstichspitze (6) entgegengesetzten Ende befestigten Nahtmaterial oder Faden (4), **dadurch gekennzeichnet, dass** die Oberfläche der Einstichspitze (6) und des sich daran anschließenden Bereichs (8) der Nadel (2) über mehr als etwa 50 % der Nadellänge aus blankem oder unbehandeltem Metall besteht, während die Oberfläche des restlichen Teils (10) der Nadel (2) bis zum Fadenansatz (12) chemisch oder elektrolytisch oder durch einen Überzug durchgehend oder mit geringfügigen Unterbrechungen mattiert oder gefärbt ist.

2. Chirurgische Nadel nach Anspruch 1, **dadurch gekennzeichnet, dass** der Bereich (10) der Nadel (2), dessen Oberfläche chemisch oder elektrolytisch oder durch einen Überzug durchgehend oder mit geringfügigen Unterbrechungen mattiert oder gefärbt ist, weniger als 25 % der Nadellänge ausmacht.

3. Chirurgische Nadel nach einem der vorhergehenden Ansprüche, **dadurch gekennzeichnet, dass** der mattierte oder gefärbte Bereich der Nadel in Ringform oder in Sprenkelform (10) mattiert oder gefärbt ist, wobei die Oberfläche zwischen den Ringen oder Sprenkeln blank oder unbehandelt ist.

## Revendications

1. Aiguille chirurgicale avec un moyen ou un fil de suture (4) attaché à l'extrémité opposée à la pointe de piqûre (6), **caractérisée en ce que** la surface de la pointe de piqûre (6) et de la zone (8) de l'aiguille (2) voisine de celle-ci, s'étendant sur plus d'environ 50 % de la longueur de l'aiguille, est constituée de métal nu ou sans traitement de surface, tandis que la surface de la section restante (10) de l'aiguille (2) a fait l'objet d'une finition mate ou d'une coloration, continue ou avec de petites interruptions, par des moyens chimiques ou électrolytiques ou par un revêtement, jusqu'à la partie d'attache de fil (12).

2. Aiguille chirurgicale selon la revendication 1, **caractérisée en ce que** la zone (10) de l'aiguille (2), dont la surface a fait l'objet d'une finition mate ou d'une coloration, continue ou avec de petites interruptions, par des moyens chimiques ou électrolytiques ou par un revêtement, représente moins de 25 % de la longueur de l'aiguille.

3. Aiguille chirurgicale selon une quelconque des revendications précédentes, **caractérisée en ce que** la zone, ayant fait l'objet d'une finition mate ou d'une coloration, de l'aiguille a fait l'objet d'une finition mate ou d'une coloration sous une forme annulaire ou sous une forme tachetée (10), la surface entre les anneaux ou les taches étant nue ou non traitée.
